(19) Europäisches Patentamt
European Patent Office
Office européen des brevets

(11) **EP 2 696 956 B1**

(12) **EUROPÄISCHE PATENTSCHRIFT**

(45) Veröffentlichungstag und Bekanntmachung des Hinweises auf die Patenterteilung:
**03.01.2018 Patentblatt 2018/01**

(21) Anmeldenummer: **12714701.5**

(22) Anmeldetag: **16.04.2012**

(51) Int Cl.:
*B01L 3/00* *(2006.01)*       *B01D 63/08* *(2006.01)*
*G01N 1/28* *(2006.01)*       *G01N 1/40* *(2006.01)*
*G01N 15/10* *(2006.01)*      *G01N 1/04* *(2006.01)*
*G01N 33/50* *(2006.01)*

(86) Internationale Anmeldenummer:
**PCT/EP2012/056872**

(87) Internationale Veröffentlichungsnummer:
**WO 2012/159822 (29.11.2012 Gazette 2012/48)**

(54) **ANORDNUNG UND VERFAHREN ZUR OPTISCHEN ANALYSE UND SPEZIFISCHEN ISOLIERUNG VON BIOLOGISCHEN PROBEN**

ARRANGEMENT AND PROCESS FOR OPTICAL ANALYSIS AND SPECIFIC ISOLATION OF BIOLOGICAL SAMPLES

DISPOSITIF ET PROCÉDÉ D'ANALYSE OPTIQUE ET DE SÉPARATION SPÉCIFIQUE D'ÉCHANTILLONS BIOLOGIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **20.05.2011 DE 102011076238**

(43) Veröffentlichungstag der Anmeldung:
**19.02.2014 Patentblatt 2014/08**

(73) Patentinhaber: **Siemens Healthcare GmbH
91052 Erlangen (DE)**

(72) Erfinder:
• **GUMBRECHT, Walter
91074 Herzogenaurach (DE)**
• **BANGERT, Joachim
91052 Erlangen (DE)**
• **FRIEDRICH, Katja
69306 Erlenbach (DE)**
• **HILTAWSKY, Karsten
58239 Schwerte (DE)**
• **PAULICKA, Peter
91341 Röttenbach (DE)**
• **STANZEL, Manfred
92334 Berching (DE)**

(74) Vertreter: **Schweitzer, Klaus
Plate Schweitzer Zounek
Patentanwälte
Rheingaustrasse 196
65203 Wiesbaden (DE)**

(56) Entgegenhaltungen:
EP-A1- 1 260 807       WO-A1-2011/092201
US-A- 3 731 806        US-A- 4 124 449
US-A- 5 321 545

• ANONYMOUS: "Nuclepore Track-Etched Membranes", WHATMAN - GE HEALTHCARE , 2009, Seiten 1-4, XP002679438, Gefunden im Internet: URL:http://www.whatman.com [gefunden am 2012-07-05]

EP 2 696 956 B1

**Beschreibung**

Die Erfindung betrifft ein Verfahren zum Nachweis von Zellen in einer flüssigen Probe sowie eine Anordnung, welche zur Durchführung des Verfahrens verwendet werden kann.

Die Mikroskopie ist ein weit verbreitetes Mittel in der Analytik. Insbesondere in den "Life-Science" Wissenschaften stellt sie ein unverzichtbares Werkzeug dar, um z.B. Gewebe und Zellen zu charakterisieren. Als "Interface" zwischen dem zu untersuchenden Medium und den abbildenden Komponenten eines Mikroskops hat sich der Objektträger etabliert. Es handelt sich dabei um ein Glasplättchen von 26 x 76 mm (ISO 8255-2) und einer Dicke von 1 bis 1.5 mm. Die Objekte werden z.B. in dünner Schicht (Gewebeschnitt, Flüssigkeitsfilm) auf den Objektträger aufgebracht und meistens mit einem Deckgläschen (häufig 18 mm x 18 mm ; 0.16 mm dick) abgedeckt.

Die Filtrationstechnik ist ebenfalls eine weitverbreitete Labor-Technik, insbesondere zur Trennung von Festkörpern verschiedener Größe, bzw. von Flüssigkeiten.

Bei der Kombination von Mikroskopie und Filtrationstechnik wird der Filterrückstand im Anschluss an den Filtrationsprozess mikroskoptechnisch untersucht. Dazu muss das Filtermedium, z.B. die Filtrations-Membran aus dem Filtrations-Gerät entnommen werden und auf den Objektträger gelegt werden. Dieser Vorgang erfordert, insbesondere bei dünnen Membranen (z.B. 10 $\mu$m Dicke, 25 mm Durchmesser), hohes experimentelles Geschick und ist sehr zeitaufwendig. Soll dieser Vorgang routinemäßig und kostengünstig z.B. in der medizinischen Diagnostik eingesetzt werden, z.B. bei der Untersuchung von Tumorzellen, die aus einer Blutprobe filtriert wurden, muss eine einfache und preisgünstige Lösung entwickelt werden, die auch von ungeschultem Personal ausgeführt werden kann. Insbesondere eine Isolation von ausgewählten einzelnen Zellen, z.B. zirkulierende Tumorzellen, (circulating tumor cells mCTCs) oder Zellverbänden für eine nachfolgende molekulardiagnostische Untersuchung wird aufgrund der wissenschaftlichen Fortschritte auf diesem Gebiet in der in Zukunft von zunehmender Bedeutung sein.

Aufgabe der vorliegenden Erfindung ist es deshalb, eine Anordnung und ein Verfahren optischen Analyse und spezifischen Isolierung von biologischen Proben anzugeben, welche in Standartverfahren mit hoher Qualität eingesetzt werden können und einfach aufgebaut, einfach handhabbar sowie kostengünstig sind. Insbesondere ist es Aufgabe der vorliegenden Erfindung eine Anordnung und ein Verfahren zur optischen Analyse und spezifischen Isolierung von biologischen Proben anzugeben, welche gut für eine anschließende mikroskopische Untersuchung geeignet sind. Dabei sollte die Anordnung in Standartgeräten mit Standardhalterungen einsetzbar sein, um z.B. lichtmikroskopische oder fluoreszenzmikroskopische Untersuchungen serienmäßig einfach und billig an dem Filtrationsrückstand durchführen zu können. Insbesondere ist es eine Aufgabe, den Einsatz in der Gewinnung und Untersuchung von (zirkulierenden) Tumorzellen (CTC) zu ermöglichen.

Die Erfindung betrifft ein Verfahren nach Anspruch 1 und eine Anordnung nach Anspruch 11. US 3,731,806 (McCORMICK), 8. Mai 1973, kann als nächstliegender Stand der Technik angesehen werden.

Bei einem Filtrationsvorgang im Sinne der Erfindung wird eine Suspension durch ein Filter, z.B. eine Filtermembran, filtriert. Dabei wird Permeat durch den Filter gedrückt und Retentat auf der Filteroberfläche(oder auch in den Poren und Hohlräumen des Filters) zurückgehalten. Bei dem Filtriervorgang gibt es daher eine vorherrschende Strömungsrichtung des Permeats durch den Filter, so dass man von einem Bereich stromaufwärts von dem Filter sprechen kann, in welchem das Retentat (welches als wesentlichen Bestandteil die Zellen enthält) zurückgehalten wird, und einem Bereich stromabwärts, in welchen das Permeat durchgedrückt wird und z.b. dort gesammelt werden kann. Unabhängig von dieser vorherrschenden Strömungsrichtung kann in Ausnahmefällen die Strömungsrichtung auch umgekehrt werden, z.B. bei einer Rückspülung des Filters.

Um das Permeat durch den Filter zu drücken, kann eine Druckdifferenz erzeugt werden, wobei dann stromaufwärts von dem Filter ein höherer Druck vorliegt als Stromabwärts. Dies kann erreicht werden durch Anlegen eines Überdrucks stromaufwärts von dem Filter, Anlegen eines Unterdurcks stromabwärts oder einer Kombination aus beiden. Um den Permeatfluss durch den Filter zu stoppen (auf Null zu reduzieren), kann eine Druckdifferenz von Null eingestellt werden. Dies ist unabhängig von der Orientierung des Filters im Raum. Für den Sonderfall, dass die Strömungsrichtung am Filter vertikal verläuft oder eine vertikale Komponente (also in Richtung oder entgegen der Erdanziehungskraft) verläuft, ist ferner zu berücksichtigen, dass die Wassersäule auf dem Filter zur Druckkdifferenz beiträgt.

Für manche Anwendungen des erfindungsgemäßen Verfahrens ist es bevorzugt, dass die strömungsrichtung der Filtration am Filter im Wesentlichen in Richtung der Erdanziehungskraft verläuft. Dadurch kommt zurückgehaltenes Retentat auf der Oberfläche des Filters zu liegen, was z.b. eine einfache Weiterverarbeitung des Retenats (der Zellen) ermöglicht.

Für bestimmte Anwendungen kann es bevorzugt sein, nicht in Richtung der Erdanziehungskraft, sondern entgegen der Erdanziehungskraft zu Filtrieren, z.B. wenn das Retentat aufschwimmt, oder um Zellen nach dem Filtrieren von der Unterseite des Filters in einem Auffanggefäß zu sammeln.

Die Erfindung betrifft ein Verfahren zum Nachweis von Zellen in einer flüssigen Probe, aufweisend folgende Schritte:

i) Filtrieren der flüssigen Probe durch eine poröse Membran, die geeignet ist, nachzuweisende Zellen zurückzuhalten, wobei zumindest ein Teilbereich eines Trägers als transparenter, strukturierter Stützkörper ausgebildet ist und wobei die Membran flächig auf dem transparenten Stützkörper angeordnet ist, derart, dass nachzuweisende Zellen auf zumindest einem Teil der Oberfläche der Membran zurückgehalten werden und dass zumindest ein Teil der Probenflüssigkeit die Membran passiert,

ii) Aufbringen eines flüssigen optischen Mediums, welches im Wesentlichen den gleichen Brechungsindex wie der Stützkörper hat, wobei das flüssige optischen Medium so aufgebracht wird, dass es die Zwischenräume zwischen der Membran und dem Stützkörper füllt,

iii) optisches Erfassen mindestens einer Teilfläche der Membran zum Detektieren nachzuweisender Zellen.

Auf dem Stützkörper kann die Membran angeordnet werden. Er dient dazu, die Membran zu stützen und den Ablauf von Filtrat zu ermöglichen. Die Membran kann auf dem Stützkörper aufliegen. Dazu können in der Oberfläche des Stützkörpers Kanäle ausgebildet sein, um einen Ablauf von Filtrat zu gewährleisten. Gemäß einer alternativen Ausführungsform können in dem Stützkörper auch Löcher oder Poren vorgesehen sein. Der Stützkörper kann als separates Teil in einer Ausnehmung des Trägers ausgebildet sein, er kann aber auch integral mit dem Träger in einem Teilbereich des Trägers ausgebildet sein.

Der Brechungsindex n gibt das Verhältnis der Vakuumlichtgeschwindigkeit c0 zur Ausbreitungsgeschwindigkeit cM des Lichts im Medium M an:

$$n = c0/cM$$

Der Brechungsindex ist eine dimensionslose Zahl. Ein im Wesentlichen gleicher Brechungsindex bedeutet, dass sich der Brechungsindex um nicht mehr als 0,1, bevorzugt nicht mehr als 0,01, stärker bevorzugt nicht mehr als 0,001 unterscheidet.

Der Träger weist bevorzugt im Wesentlichen die Abmessungen eines Mikroskopie-Objektträgers auf.

Die Membran und der Stützkörper können z.B. eine im Wesentlichen quadratische, runde oder elliptische Grundfläche aufweisen.

Durch die erfindungsgemäße Verwendung eines optischen Mediums, welches auf das Material des Stützkörpers abgestimmt ist, wird die optische Erfassung wesentlich verbessert.

Bevorzugt sind Membran und Stützkörper so gewählt, dass sie ebenfalls im Wesentlichen den gleichen Brechungsindex haben.

Gemäß einem Aspekt der Erfindung werden die nachzuweisenden Zellen vor oder nach dem Filtrieren markiert werden mit einem ersten Mittel zum Markieren der nachzuweisenden Zellen, welches einen ersten Marker enthält.

Geeignete Mittel zum Markieren umfassen Marker die unspezifisch oder spezifisch Zellen anfärben können. Unspezifische Marker können z.B. Farbstoffe sein, die Proteine, Nukleinsäuren oder andere Zellbestandteile anfärben. Spezifische Marker können z.B. Antikörper, Sondenoligonucleotide, Peptide oder andere Moleküle sein, die spezifisch an Proteine, Nukleinsäuresequenzen oder andere Zellspezifische Strukturen binden.

Der Marker kann mit einer nachweisbaren Markierung direkt markiert sein, z.B. chromogene Farbstoffe, Fluoreszenzfarbstoffe, Isotopenmarkierung o.ä.. Alternativ kann der Marker auch über ein sekundäres Nachweisreagenz (z.B. Sekundärantiköper oder Enzym-Substrat-System) nachgewiesen werden.

Gemäß einem bevorzugten Aspekt der Erfindung werden bei dem Verfahren nach dem Filtrieren die Zellen auf der Membran mit einem Farbstoff durch Inkubation mit einer entsprechenden Prozessflüssigkeit angefärbt. Dazu können Farbstoffe gewählt werden, die Zellen oder Zellbestandteile anfärben und aus Cytologie und Histologie bekannt sind. Dies können Lebend- oder Totfarbstoffe sein, Farbstoffe, die spezifisch Zellkerne oder andere Organellen anfärben oder die spezifisch bestimmte Zellkomponenten, z.B. Nukleinsäuren oder Proteine anfärben. Bekannte Zellfarbstoffe sind, z.B. Trypanblau, DAPI und ähnliche.

Die nachzuweisenden Zellen können auf der Membran ungefärbt oder gefärbt auch mikroskopisch analysiert werden.

Die Membran liegt flächig bevorzugt auf dem transparenten Stützkörper (3) auf und bedeckt diesen zumindest teilweise oder auch vollständig.

Gemäß einem Aspekt der Erfindung ist der Träger ein Objektträger für die Mikroskopie, welcher aus Glas oder aus Plastik, insbesondere Cyclischem Olefin-Copolymer (COC) oder Polykarbonat, ausgebildet ist. Ein bevorzugtes COC ist unter dem Handelsnamen TOPAS ®bekannt.

Gemäß einem Aspekt der Erfindung ist der Stützkörper porös, und aus Plastik, Cyclischem Olefin-Copolymer (COC) oder Polykarbonat, insbesondere aus dem selben Material ausgebildet wie der Träger ausgebildet. Gemäß einem Aspekt der Erfindung sind der Träger und der Stützkörper integral aus einem Körper ausgebildet und die Membran bedeckt die Ausnehmung im Träger vollständig und ist insbesondere flach, insbesondere planparallelauf dem Stützkörper im Bereich der Ausnehmung aufliegend.

Gemäß einem Aspekt der Erfindung umfasst der Stützkörper auf einer der Filter-Membran zugewandten Seite ausgebildete Kanäle umfasst, welche in fluidischem Kontakt mit der Filter-Membran ausgebildet sind. Die Kanäle können zu Öffnungen im Stützkörper und/oder dem Träger ausgebildet sind, wodurch Flüssigkeit von der Membran weg durch den Stützkörper bzw. Träger ablaufen oder abgesaugt werden kann. Die Öffnungen können z.B. als senkrechte Bohrungen von der Oberseite zur Unterseite des Stützkörper bzw. Trägers ausgebildet sein. Die Membran und der Stützkörper können z.B. eine im Wesentlichen quadratische, runde oder elliptische Grundfläche aufweisen. Die Öffnungen können in regelmäßigen Abständen am Rand der Grundfläche vorhanden sein. Diese bevorzugte Anordnung ermöglicht einen Gleichmäßigen Ablauf des Filtrats. Gleichzeitig befindet deckt die Membran im Wesentlichen den Stützkörper ab, so dass bei Auffüllen der Zwischenräume zwischen Membran und Stützkörper mit dem optischen Medium und auch bei einem eventuellen Auffüllen der Membranporen mit dem Medium eine optische Einheit mit einheitlichem Brechundsindex entsteht, auf welcher man besonders gut mikroskopieren kann. Alternativ können die Öffnungen auch im Wesentlichen gleichmäßig über die Grundfläche des Stützkörpers verteilt sein.

Gemäß einem Aspekt der Erfindung bilden die Membran und der Stützkörper eine gemeinsame Kontaktfläche mit einer Vielzahl von gemeinsamen, in einer plan-parallelen, flachen Ebene der Kontaktfläche liegenden Kontaktstellen, wobei insbesondere die Membran einen maximalen Abstand von dem Stützkörper von weniger als 100 μm aufweist. Vorzugsweise liegt die Membran an den Kontaktstellen auf.

Gemäß einem Aspekt der Erfindung ist die Filter-Membran (2) eine Track Etched Filter-Membran, welche aus einer Polykarbonatfolie aufgebaut ist und Poren mit einem Durchmesser von 2-100μm, insbesondere 5-20μm, bevorzugt 5-10 stärker bevorzugt ca. 8μm umfasst.

Gemäß einem Aspekt der Erfindung kann eine nachzuweisende Zelle isoliert werden. Die isolierte Zelle kann dann näher charakterisiert werden oder weiteren funktionellen Untersuchungen unterzogen werden.

Gemäß einem Aspekt der Erfindung kann die nachzuweisende Zelle durch Laser-Mikrodissektion isoliert werden. Bevorzugt können optische Detektion und anschließende Laser-Mikrodissektion im selben Gerät in aufeinander folgenden Arbeitsschritten erfolgen.

Die Laser-Mikrodissektion ist eine mikroskopische Technik zur lasergestützten Mikrodissektion von Geweben und Zellen. Hierbei kann aus einem Gewebeschnitt mithilfe eines Lasers (z.B. ein Infrarot- oder Ultraviolett-Laser) eine Einzelzelle oder eine gewünschte Region ausgeschnitten werden. Es ist möglich, die Zelle mit dem Flächenabschnitt der Membran, auf dem sie liegt, gemeinsam "auszuschneiden". Die reine Zelle wird dann entweder isoliert, indem sie unter dem Einfluss der Schwerkraft in ein Reaktionsgefäss fällt, oder sie wird gegen die Schwerkraft in ein solches katapultiert oder wird indirekt, von dem adhesiven Deckel des Reaktionsgefässes abgehoben. Gemäß einem Aspekt der Erfindung ist die nachzuweisende Zelle eine Tumorzelle.

Die Erfindung betrifft ferner eine Anordnung zum Nachweis von Zellen in einer flüssigen Probe, aufweisend einen Träger, die geeignet ist, nachzuweisende Zellen zurückzuhalten, wobei die Membran flächig auf einem strukturierten Stützkörper angeordnet ist, wobei der Stützkörper in einer Ausnehmung des Trägers angeordnet und/oder ausgebildet ist und ein flüssiges optisches Medium, welches im Wesentlichen den gleichen Brechungsindex wie der Stützkörper hat, die Zwischenräume zwischen der Membran und dem Stützkörper füllt, wobei zumindest der Stützkörper transparent ist.

Als Anordnung im Sinne der Erfindung wird hier eine Zusammenstellung ("Kit of Parts") aus Träger, Membran und dem flüssigen optischen Medium angesehen. Die Membran kann mit dem Träger und/oder dem Stützkörper verbunden sein, z.B. durch eine Verklebung, Verschweißung, eine Klemmverbindung oder ähnliches. Gemäß eine Bevorzugten Ausführungsform deckt die Membran den Stützkörper ab und ist, z.B. mit punktförmigen Verschweißungen, am Rand mit dem Träger verbunden. Es ist bevorzugt, dass der Träger ein Objektträger für die Mikroskopie ist, welcher aus Glas oder aus Plastik, insbesondere Polykarbonat ausgebildet ist, und/oder dass der Stützkörper porös, aus Plastik, insbesondere Polykarbonat ausgebildet ist.

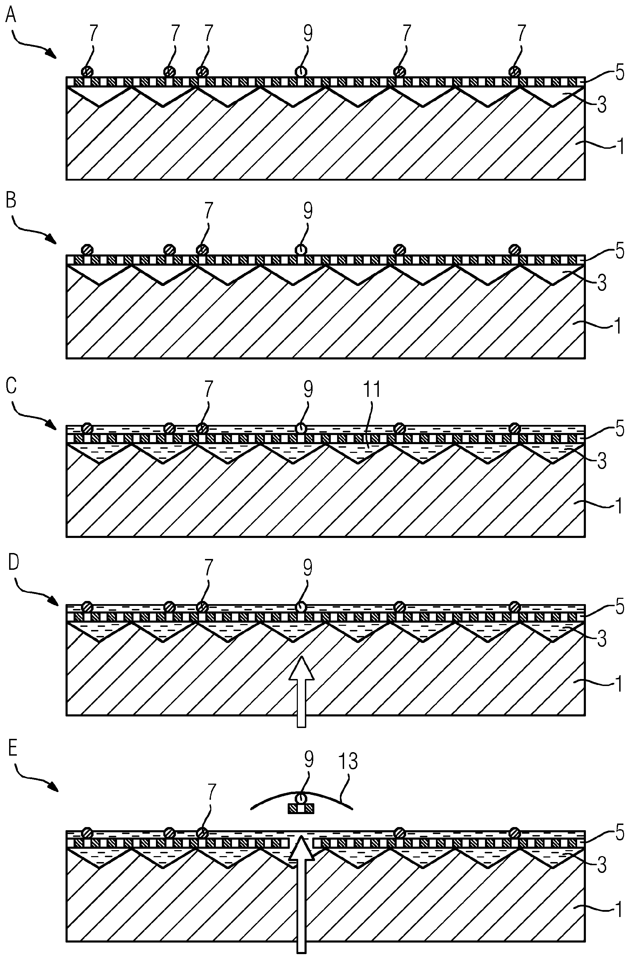

Die Erfindung wird im Folgenden beispielhaft beschrieben anhand der Figur, welche schematisch eine Ausführungsform des erfindungsgemäßen Verfahrens darstellt. In Figur 1 ist in einer Schnittansicht ein flacher Stützkörper 1 gezeigt, der z.B. aus COC, z.B. insbesondere aus dem COC mit Handelnamen TOPAS, besteht. In dem Stützkörper 1 sind auf der Oberseite, welche einer Membran 5 zugewandt ist, Kanäle 3 ausgebildet. Diese Kanäle durchziehen die Oberfläche des Stützkörpers und können in (durch den Stützkörper hindurch verlaufende) Bohrungen oder Öffnungen münden, durch welche Flüssigkeiten von der Oberseite des Stützkörpers abgesaugt werden können.

Anstatt Kanäle vorzusehen, ist es auch möglich, auf der Oberseite des Stützkörpers Erhebungen vorzusehen, die z.B. als Pyramiden- oder Kegelstümpfe ausgebildet sind und deren Oberflächen in einer Ebene liegen, wodurch eine Auflagefläche oder Kontaktfläche definiert wird, auf der die Membran 5 aufliegt. Gemäß einer bevorzugten Ausführungsform ist die Oberfläche des Stützkörpers als hexagonale Packung von 150 μm hohen Kegelstümpfen mit einem Rastermaß von 300 μm aufgebaut. Die Zwischenräume der Kegelstümpfe definieren Kanöle in welchen die Flüssigkeit ablaufen kann und die Stumpfflächen der Kegelstümpfe definieren eine Auflagefläche für die Membran.

Ein Teilbereich des Trägers ist als Stützkörper ausgebildet. Der Träger weist vorzugsweise die Abmessungen eines Objektträgers auf, wie er in der Mikroskopie verwendet wird.

Die Porengröße der Membran ist so gewählt, dass nachzuweisende Zellen 9 die Membran nicht passieren können.

Die Probe wird nun in einem ersten Schritt A der Figur 1 filtriert, so dass nachzuweisende Zellen 9 auf der Membran zu liegen kommen.

Die Membran weist eine Porengröße von 0,1 bis 200μm auf. Dadurch können Zellen zurückgehalten werden, während Zellfragmente, Thrombozyten und kleinere Festbestandteile der Probe durch das Filter (die Membran) hindurch gehen.

Bevorzugt weist die Membran eine Porengröße von 2 bis 50$\mu$m, stärker bevorzugt 5 bis 20$\mu$m, noch stärker bevorzugt 5 bis 10$\mu$m, auf. Porengrößen der Größenbereiche 2 bis 50$\mu$m, 5 bis 20$\mu$m oder insbesondere 5 bis10 $\mu$m bieten den Vorteil, dass die Zellen davon zurückgehalten werden, jedoch teilweise in den Poren haften bleiben und so besonders gut auf der Membran haften und für weitere Analysen zur Verfügung stehen. Als besonders geeignet hat sich eine Porengröße von ca. 8 $\mu$m erwiesen.

Die von der Probenflüssigkeit zu trennenden Zellen werden dadurch getrennt, dass Sie auf der Oberfläche einer Filtermembran verbleiben, die impermeabel für die zu trennenden Feststoffe (z.B. Zellen) ist, jedoch permeabel für das Umgebungsmedium und auch für den zu trennenden Feststoff (Zellen) verunreinigende Feststoffe (z.B. Zellfragmente) ist.

In einem optionalen Schritt B der Figur 1 können nachzuweisende Zellen 9 markiert werden, damit sie besser von anderen Zellen 7 unterschieden werden können. Dies kann z.B. mit einer immunhistochemischen Färbung bestimmter Antigene erfolgen. Falls Tumorzellen nachgewiesen werden sollen, steht eine große Anzahl bekannter Tumorantigene zur Verfügung, die mit einem entsprechenden Antikörper markiert werden können. So ist es möglich, spezifisch Tumorzellen nachzuweisen. Entsprechende Antikörper und Färberprotokolle sind dem Fachmann bekannt.

Die folgende Liste zeigt einige bevorzugte zellspezifische Antigene:

- Alpha-1-Fetoprotein (AFP) bei Leberzellkarzinom und gonadalen und extragonadalen Keimzelltumoren
- Bence-Jones-Protein beim Multiplen Myelom
- Beta-HCG (beta-Untereinheit des humanen Choriongonadotropin) bei Keimzelltumoren des Ovars und nichtseminomatösem Tumoren des Hodens
- CA 15-3 beim Brustkrebs (Mammakarzinom) oder Eierstockkrebs (Ovarialkarzinom)
- CA 19-9 und CA 50 beim Bauchspeicheldrüsenkrebs (Pankreaskarzinom)
- CA-125 beim Eierstockkrebs (Ovarialkarzinom)
- Calcitonin (humanes Calcitonin, hCT), beim medullären Schilddrüsenkarzinom
- Carcino-Embryonales Antigen (CEA) bei Darmkrebs, Pankreaskarzinom sowie Adenokarzinom der Lunge
- Cytokeratin-21-Fragment (CYFRA 21-1) und Serpin B4 (SCC) bei allen Varianten des Lungenkrebses (Bronchialkarzinoms)
- HER-2/neu
- HPV-Antikörper bzw. HPV-Antigene
- Homovanillinsäure beim Neuroblastom
- 5-Hydroxyindolessigsäure beim Karzinoid

- Katecholamine, Vanillinmandelsäure beim Phäochromozytom
- Laktat-Dehydrogenase (LDH) bei Keimzelltumoren
- Laktat-Dehydrogenase Isoenzym 1 (LDH-1) bei Keimzelltumoren; eine routinemäßige Bestimmung wird in den gängigen Leitlinien jedoch noch nicht empfohlen
- MAGE Antigene
- Metanephrine beim Phäochromozytom
- MUC1 beim nicht kleinzelligen Bronchialkarzinom (NSCLC) oder beim Mammakarzinom
- NSE beim kleinzelligen Bronchialkarzinom (SCLC), Neuroblastom sowie seminomatösen Keimzelltumoren
- Plazentare alkalische Phosphatase (PLAP) bei seminomatösen Keimzelltumoren
- PSA beim Prostatakrebs (Prostatakarzinom)
- Thyreoglobulin (Tg) in jeder Konzentration beim papillären oder follikulären Schilddrüsenkarzinom
- Thymidinkinase
- Cytokeratine, z.B. Cytokeratin 8, 18, 19

Durch das Markieren kann somit ein spezifischer Zelltyp erkannt werden. Damit ist es z.B. möglich in einer Blutprobe nachzuweisende Tumorzellen von Leukozyten zu unterscheiden. Alternativ oder zusätzlich können Zellen aber auch mit einem unspezifischen Farbstoff (der nicht zwischen verschieden Zelltzpen differenziert) markiert werden, z.b. einen Lebendfarbstoff (z.B. Fluorescein) oder einen Totfarbstoff (z.B. Trzpanblau). Dadurch können z.B. lebende bzw. tote Zellen für die weitere Analyse selektiert werden.

In einem weiteren Schritt C der Figur 1 wird ein flüssiges optisches Medium 11 aufgebracht, welches im Wesentlichen den gleichen Brechungsindex wie der Stützkörper hat.

Das flüssige optische Medium 11 wird so aufgebracht, dass es die Zwischenräume zwischen der Membran 5 und dem Stützkörper 1 füllt, welche z.B. durch die Kanalstruktur 3 im Stützkörper 1 definiert sind. Ferner füllt das flüssige optische Medium 11 auch die Poren der Membran 5 und bedeckt bevorzugt auch die Oberfläche der Membran 5 und die darauf liegenden Zellen in dünner Schicht.

Dadurch werden die optischen Abbildungseigenschaften der Anordnung aus Stützkörper 1 und Membran 5 deutlich verbessert, was eine bessere und vereinfachte optische Detektion von Zellen auf der Membran ermöglicht. Dies wird erleichtert durch die Bereitstellung guter Transparenz des Membranträger-Kunststoffs im UV-A Bereich (z.B. 337nm).

Vor der Detektion erfolgt die Füllung der Fluidik-Kanäle zwischen Stützkörper 1 und Membran 5 durch ein flüssiges optisches Medium 11 mit zumindest annähernd gleichem Brechungsindex wie dem des Membranträgers (n=1,533) derart, dass die biologische Probe auf der Membranoberfläche noch in Kontakt mit dem optischen Medium steht. Die Herstellung eines entsprechenden optischen Mediums mit einem vorbestimmten Brechungsindex ist dem Fachmann bekannt. Der Brechungsindex des optischen Mediums (z.B. einer Zuckerlösung) kann mit einem Refraktometer bestimmt und die Lösung durch Verdünnung entsprechend eingestellt werden. Bevorzugt sind die Dampfdrücke der Komponenten des optischen Mediums vernachlässigbar.

Bevorzugt erfolgt in Schritt D der Figur 1 die optische Analyse der Zellen von der Rückseite her, durch den ca. 1mm dicken Träger-Kunststoff. Dies kann z.B. mit einem Inversmikroskop erfolgen. Der Pfeil deutet hierbei die Richtung an, aus welcher die Zelle 9 detektiert wird.

Anschliessend kann in einem optionalen Schritt E der Figur 1 eine erfasste nachzuweisende Zelle mit Hilfe von Laserdissektion isoliert werden.

Der Pfeil deutet hierbei die Richtung an, aus welcher die Zelle 9 (und evtl. eine Teilfläche der Membran, auf der die Zelle liegt) mit einem Laserstrahl aus der Membran gelöst und von einem Probengefäß 13 aufgefangen wird. Die isolierte Zelle kann weiteren Tests unterzogen werden.

Das Erfindungsgemäße Verfahren eignet sich besonders gut für diese Kombination mit der Laserdissektion, da der Einsatz des optischen Mediums besonders präzises Arbeiten ermöglicht.

Alternativ kann die Zelle auch auf der Membran verbleiben und dort weiteren Tests unterzogen werden, z.B. in einem EPISPOT oder ELISPOT (Enzyme Linked Immuno Spot Technique) - Verfahren. Hierbei werden auf der Membran immunchemisch Stoffe nachgewiesen, die von der Zelle sezerniert werden.

**Patentansprüche**

1. Verfahren zum Nachweis von Zellen in einer flüssigen Probe, aufweisend folgende Schritte:

   i) Filtrieren der flüssigen Probe durch eine poröse Membran, die geeignet ist, nachzuweisende Zellen (9) zurückzuhalten, wobei zumindest ein Teilbereich eines Trägers als transparenter, strukturierter Stützkörper (1) ausgebildet ist und wobei die Membran (5) flächig auf dem transparenten Stützkörper (1) angeordnet ist, derart, dass nachzuweisende Zellen (9) auf zumindest einem Teil der Oberfläche der Membran (5) zurückgehalten werden und dass zumindest ein Teil der Probenflüssigkeit die Membran (5) passiert,
   ii) Aufbringen eines flüssigen optischen Mediums (11), dessen Brechungsindex sich um nicht mehr als 0.1 vom Brechungsindex des Stützkörpers unterscheidet, wobei das flüssige optische Medium (11) so aufgebracht wird, dass es die durch die Strukturierung des Stützkörpers bestehenden Zwischenräume (3) zwischen der Membran (5) und dem Stützkörper (1) füllt,
   iii) optisches Erfassen mindestens einer Teilfläche der Membran (5) zum Detektieren nachzuweisender Zellen (9).

2. Verfahren nach Anspruch 1, wobei die nachzuweisenden Zellen (9) vor oder nach dem Filtrieren markiert werden mit einem ersten Mittel zum Markieren der nachzuweisenden Zellen, welches einen ersten Marker enthält.

3. Verfahren nach einem der vorangehenden Ansprüche, wobei der Träger ein Objektträger für die Mikroskopie ist, welcher aus Glas oder aus Plastik, insbesondere COC ausgebildet ist, und/oder dass der Stützkörper (1) porös, aus Plastik, insbesondere aus dem gleichen Material ausgebildet ist.

4. Verfahren nach einem der vorhergehenden Ansprüche, wobei der Träger und der Stützkörper (1) integral aus einem Körper ausgebildet sind und die Membran (5) den Stützkörper (1) vollständig bedeckt und insbesondere flach auf dem Stützkörper aufliegt.

5. Verfahren nach einem der vorhergehenden Ansprüche, wobei der Stützkörper (1) auf einer der Filter-Membran zugewandten Seite ausgebildete Kanäle (3) umfasst, welche in fluidischem Kontakt mit der Membran (5) ausgebildet sind.

6. Verfahren nach einem der vorhergehenden Ansprüche, wobei die Membran (5) und der Stützkörper (1) eine Vielzahl von gemeinsamen, in einer plan-parallelen, flachen Ebene, der Kontaktfläche liegende Kontaktstellen aufweisen, wobei insbesondere die Membran (5) einen maximalen Abstand von der ebenen Kontaktfläche von weniger als $100\mu m$ aufweist.

7. Verfahren nach einem der vorhergehenden Ansprüche, wobei die Membran (5) eine Track Etched Filter-Membran ist, welche aus einer Polykarbonatfolie aufgebaut ist und Poren mit einem Durchmesser von $2\mu m$ - $100\mu m$, insbesondere 5-10 $\mu m$ umfasst.

8. Verfahren nach einem der vorhergehenden Ansprüche, wobei mindestens eine nachzuweisende Zelle (9)isoliert wird.

9. Verfahren nach Anspruch 8, wobei die nachzuweisende Zelle (9) durch Laser-Mikrodissektion isoliert wird.

10. Verfahren nach einem der Ansprüche 8 und 9, wobei die nachzuweisende Zelle (9) eine Tumorzelle ist.

11. Anordnung zum Nachweis von Zellen in einer flüssigen Probe, aufweisend einen Träger, eine poröse Membran (5), die geeignet ist, nachzuweisende Zellen (9) zurückzuhalten;

wobei zumindest ein Teilbereich des Trägers als strukturierter Stützkörper (1) ausgebildet ist, wobei die Membran flächig auf dem Stützkörper (1) angeordnet ist, und wobei durch die Strukturierung des Stützkörpers Zwischenräume (3) zwischen der Membran und dem Stützkörper (1) bestehen; wobei zumindest der Stützkörper transparent ist, und wobei ein flüssiges optisches Medium (11), dessen Brechungsindex sich um nicht mehr als 0.1 vom Brechungsindex des Stützkörpers unterscheidet, die genannten Zwischenräume (3) füllt.

12. Anordnung nach Anspruch 11, wobei der Träger und der Stützkörper (1) integral aus einem Körper ausgebildet sind und die Membran (5) den Stützkörper vollständig bedeckt und insbesondere flach auf dem Stützkörper im Bereich einer Ausnehmung aufliegt.

13. Anordnung nach Anspruch 11 oder 12, wobei der Stützkörper (1) auf einer der Membran (5) zugewandten Seite ausgebildete Kanäle (3) umfasst, welche in fluidischem Kontakt mit der Filter-Membran ausgebildet sind.

14. Anordnung nach einem der Ansprüche 11 bis 13, wobei die Membran (5) und der Stützkörper (1) eine Vielzahl von gemeinsamen, in einer plan-parallelen, flachen Ebene, die eine Kontaktfläche bildet, liegende Kontaktstellen aufweisen, wobei insbesondere die Membran (5) einen maximalen Abstand von der ebenen Kontaktfläche von weniger als 100 µm aufweist.

15. Anordnung nach Anspruch 14, wobei der Stützkörper im Bereich des Kontaktes zwischen der Filter-Membran und dem Stützkörper in Form von Stegen, insbesondere Stegen mit dreieckigem Querschnitt, mit einer Breite im Bereich von 50 µm bis 500 µm, in Form von Kegelstümpfen, in Form von Pyramidenstümpfen und/oder in Form von Säulen ausgebildet ist.

16. Anordnung nach einem der Ansprüche 11 bis 15, wobei die Membran (5) eine Track Etched Filter-Membran ist, welche aus einer Polykarbonatfolie aufgebaut ist und Poren mit einem Durchmesser von 2-100 Mikrometern, insbesondere 8 µm umfasst und eine Löcherdichte von $10^5$ Poren pro Quadratzentimeter aufweist.

17. Anordnung nach einem der Ansprüche 11 bis 16, wobei der Träger ein Objektträger für die Mikroskopie ist, welcher aus Glas oder aus Plastik, insbesondere COC ausgebildet ist, und/oder dass der Stützkörper (1) porös, aus Plastik, insbesondere aus dem selben Material ausgebildet ist.

**Claims**

1. Process for detection of cells in a liquid sample, comprising the following steps:

   i) filtration of the liquid sample through a porous membrane that is suitable for retaining cells (9) to be detected, wherein at least one subregion of a support is configured as a transparent, structured supporting body (1) and wherein the membrane (5) is arranged in a flat configuration on the transparent supporting body (1) in such a way that cells (9) to be detected are retained on at least part of the surface of the membrane (5) and that at least part of the sample liquid passes through the membrane (5),
   ii) application of a liquid optical medium (11) having a refractive index which differs by no more than 0.1 from the refractive index of the supporting body, wherein the liquid optical medium (11) is applied in such a manner that it fills the interstices (3) that result from the structuring of the supporting body, between the membrane (5) and the supporting body (1),
   iii) optical measurement of at least a subarea of the membrane (5) in order to detect cells (9) to be detected.

2. Process according to Claim 1, wherein the cells (9) to be detected are labeled before or after filtration with a first means for labeling the cells to be detected, which contains a first marker.

3. Process according to one of the preceding claims, wherein the support is a slide for microscopy, which is made of glass or plastic, especially COC, and/or that the supporting body (1) is porous, made of plastic, especially of the same material.

4. Process according to one of the preceding claims, wherein the support and the supporting body (1) are made integrally from a single body and the membrane (5) completely covers the supporting body (1) and especially lies flat on the supporting body.

5. Process according to one of the preceding claims, wherein the supporting body (1) has channels (3) formed on a side facing the filter membrane, said channels being configured in fluid contact with the membrane (5).

6. Process according to one of the preceding claims, wherein the membrane (5) and the supporting body (1) have a plurality of mutual contact points lying in a plane-parallel, flat plane of the contact surface, wherein especially the membrane (5) has a maximum distance from the planar contact surface of less than 100 µm.

**7.** Process according to one of the preceding claims, wherein the membrane (5) is a Track Etched filter membrane that is composed of a polycarbonate film or and has pores with a diameter of 2 $\mu$m-100 $\mu$m, especially 5-10 $\mu$m.

**8.** Process according to one of the preceding claims, wherein at least one cell to be detected (9) is isolated.

**9.** Process according to Claim 8, wherein the cell (9) to be detected is isolated by laser microdissection.

**10.** Process according to one of Claims 8 and 9, wherein the cell to be detected (9) is a tumor cell.

**11.** Arrangement for detection of cells in a liquid sample, comprising a support, a porous membrane (5) that is suitable for retaining cells (9) to be detected; wherein at least one subregion of the support is configured as a structured supporting body (1), wherein the membrane is arranged in a flat configuration on the supporting body (1), and wherein interstices (3) between the membrane and the supporting body (1) result from the structuring of the supporting body; wherein at least the supporting body is transparent, and wherein a liquid optical medium (11), having a refractive index which differs by no more than 0.1 from the refractive index of the supporting body, fills the said interstices (3).

**12.** Arrangement according to Claim 11, wherein the support and the supporting body (1) are made integrally from a single body and the membrane (5) completely covers the supporting body and especially lies flat on the supporting body in the area of a recess.

**13.** Arrangement according to Claim 11 or 12, wherein the supporting body (1) has channels (3) formed on a side facing the membrane (5), said channels being configured in fluid contact with the filter membrane.

**14.** Arrangement according to one of Claims 11 to 13, wherein the membrane (5) and the supporting body (1) have a plurality of mutual contact points lying in a plane-parallel, flat plane, which forms a contact surface, wherein especially the membrane (5) has a maximum distance from the planar contact surface of less than 100 $\mu$m.

**15.** Arrangement according to Claim 14, wherein the supporting body in the region of the contact between the filter membrane and the supporting body is configured in the form of bars, especially bars with triangular cross section, with a width in the range of 50 $\mu$m to 500 $\mu$m, in the form of truncated cones, in the form of truncated pyramids and/or in the form of columns.

**16.** Arrangement according to one of Claims 11 to 15, wherein the membrane (5) is a Track Etched filter membrane that is composed of a polycarbonate film and has pores with a diameter of 2-100 micrometers, especially 8 $\mu$m, and a pore density of $10^5$ pores per square centimeter.

**17.** Arrangement according to one of Claims 11 to 16, wherein the support is a slide for microscopy which is made of glass or plastic, especially COC, and/or that the supporting body (1) is porous, made of plastic, especially of the same material.

**Revendications**

**1.** Procédé servant à démontrer la présence de cellules dans un échantillon liquide, présentant des étapes qui suivent :

i) la filtration de l'échantillon liquide à travers une membrane poreuse qui est adaptée pour retenir des cellules (9) dont la présence est à démontrer, dans lequel au moins une zone partielle d'un support est réalisée sous la forme d'un corps de soutien (1) transparent, structuré et dans lequel la membrane (5) est disposée à plat sur le corps de soutien (1) transparent de telle manière que des cellules (9) dont la présence est à démontrer sont retenues sur au moins une partie de la surface de la membrane (5) et qu'au moins une partie du liquide d'échantillon traverse la membrane (5) ;
ii) l'application d'un milieu (11) optique liquide, dont l'indice de réfraction ne diffère pas de plus de 0,1 de l'indice de réfraction du corps de soutien, dans lequel le milieu (11) optique liquide est appliqué de telle sorte qu'il remplit les espaces intermédiaires (3) existants du fait de la structuration du corps de soutien situés entre la membrane (5) et le corps de soutien (1) ;
iii) la détection optique d'au moins une surface partielle de la membrane (5) aux fins de la détection de cellules (9) dont la présence est à démontrer.

**2.** Procédé selon la revendication 1, dans lequel les cellules (9) dont la présence est à démontrer sont marquées avant ou après la filtration à l'aide d'un premier moyen servant à marquer les cellules dont la présence est à démontrer, lequel moyen contient un premier marqueur.

**3.** Procédé selon l'une quelconque des revendications précédentes, dans lequel le support est un support d'objet pour la microscopie, lequel est réalisé à partir de verre ou à partir de plastique, en particulier de COC, et/ou que le corps de soutien (1) est réalisé poreux, à partir de plastique, en particulier à partir du même matériau.

4. Procédé selon l'une quelconque des revendications précédentes, dans lequel le support et le corps de soutien (1) sont réalisés intégralement à partir d'un corps et la membrane (5) couvre en totalité le corps de soutien (1) et repose en particulier à plat sur le corps de soutien.

5. Procédé selon l'une quelconque des revendications précédentes, dans lequel le corps de soutien (1) comprend des canaux (3) réalisés sur un côté tourné vers la membrane à filtre, lesquels sont réalisés en contact fluidique avec la membrane (5).

6. Procédé selon l'une quelconque des revendications précédentes, dans lequel la membrane (5) et le corps de soutien (1) présentent une pluralité de points de contact communs, se trouvant dans un plan plat plan-parallèle de la surface de contact, dans lequel en particulier la membrane (5) présente une distance maximale par rapport à la surface de contact plane inférieure à 100 μm.

7. Procédé selon l'une quelconque des revendications précédentes, dans lequel la membrane (5) est une membrane à filtre « track etched », qui est élaborée à partir d'un film de polycarbonate et qui comprend des pores présentant un diamètre de 2 μm - 100 μm, en particulier de 5 - 10 μm.

8. Procédé selon l'une quelconque des revendications précédentes, dans lequel au moins une cellule (9) dont la présence est à démontrer est isolée.

9. Procédé selon la revendication 8, dans lequel la cellule (9) dont la présence est à démontrer est isolée par une microdissection au laser.

10. Procédé selon l'une quelconque des revendications 8 et 9, dans lequel la cellule (9) dont la présence est à démontrer est une cellule tumorale.

11. Ensemble servant à démontrer la présence de cellules dans un échantillon liquide, présentant un support, une membrane (5) poreuse, qui est adaptée pour retenir des cellules (9) dont la présence est à démontrer, dans lequel
au moins une zone partielle du support est réalisée sous la forme d'un corps de soutien (1) structuré, dans lequel la membrane est disposée à plat sur le corps de soutien (1), et dans lequel des espaces intermédiaires (3) situés entre la membrane et le corps de soutien (1) sont existants du fait de la structuration du corps de soutien,
dans lequel au moins le corps de soutien est transparent et dans lequel un milieu (11) optique liquide, dont l'indice de réfraction ne diffère pas de plus de 0,1 de l'indice de réfraction du corps de soutien, remplit lesdits espaces intermédiaires (3).

12. Ensemble selon la revendication 11, dans lequel le support et le corps de soutien (1) sont réalisés intégralement à partir d'un corps et la membrane (5) couvre en totalité le corps de soutien et en particulier repose à plat sur le corps de soutien dans la zone d'un évidement.

13. Ensemble selon la revendication 11 ou 12, dans lequel le corps de soutien (1) comprend des canaux (3) réalisés sur un côté tourné vers la membrane (5), lesquels sont réalisés en contact fluidique avec la membrane à filtre.

14. Ensemble selon l'une quelconque des revendications 11 à 13, dans lequel la membrane (5) et le corps de soutien (1) présentent une pluralité de points de contact communs, situés dans un plan plat plan parallèle, qui forme une surface de contact, dans lequel en particulier la membrane (5) présente une distance maximale par rapport à la surface de contact inférieure à 100 μm.

15. Ensemble selon la revendication 14, dans lequel le corps de soutien est réalisé dans la zone du contact entre la membrane à filtre et le corps de soutien sous la forme d'entretoises, en particulier d'entretoises présentant une section transversale triangulaire, une largeur située dans la plage allant de 50 μm à 500 μm, sous la forme de cônes tronqués, sous la forme de pyramides tronquées et/ou sous la forme de colonnes.

16. Ensemble selon l'une quelconque des revendications 11 à 15, dans lequel la membrane (5) est une membrane à filtre « track etched », qui est élaborée à partir d'un film de polycarbonate et qui comprend des pores présentant un diamètre de 2 - 100 micromètres, en particulier de 8 μm et qui présente une densité de trous de $10^5$ pores par centimètre carré.

17. Ensemble selon l'une quelconque des revendications 11 à 16, dans lequel le support est un support d'objet pour la microscopie, qui est réalisé à partir de verre ou à partir de plastique, en particulier de COC, et/ou le corps de soutien (1) est réalisé poreux à partir de plastique, en particulier à partir du même matériau.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 3731806 A